# EUROPEAN PATENT SPECIFICATION

(11) **EP 1 005 862 B1**
(45) Date of publication and mention of the grant of the patent: **21.07.2004**
(21) Application number: 99913707.8
(22) Date of filing: 19.04.1999
(51) Int. Cl.: A61K 31/35, A61P 11/02, A61P 11/04, A61P 11/08, A61P 15/02, A61P 31/04

(54) **THE USE OF TEA POLYPHENOLS FOR THE PREPARATION OF ANTI-CHLAMYDIA AGENTS**
DIE VERWENDUNG VON TEE-POLYPHENOLEN ZUR HERSTELLUNG VON ANTI-CHLAMYDIA MITTELN
EXTRAITS DU THE POLYPHENOLIQUES DESTINEES AU TRAITEMENT DE LA CHLAMIDIE

(30) Priority: 21.04.1998 JP 12539298
(43) Date of publication of application: 07.06.2000
(73) Proprietor: Mitsui Norin Co., Ltd., Tokyo 103-8331 (JP)
(72) Inventor: YAMAZAKI, Tsutomu, Nerima-ku Tokyo 179-0081 (JP); HARA, Yukihiko, Fujieda-shi Shizuoka 426-0078 (JP)
(74) Representative: VOSSIUS & PARTNER
(86) International application number: PCT/JP1999/002063
(87) International publication number: WO 1999/053916

(56) References cited:
- DE-A- 3 603 227
- JP-A- 10 017 484
- US-A- 5 104 901
- CHEMICAL ABSTRACTS, vol. 129, no. 18, 1998 Columbus, Ohio, US; abstract no. 235671, XP002160130 & PATENT ABSTRACTS OF JAPAN vol. 1998, no. 14, 31 December 1998 (1998-12-31) & JP 10 257856 A (MITSUI NORIN KK), 29 September 1998 (1998-09-29)
- PATENT ABSTRACTS OF JAPAN vol. 1998, no. 11, 30 September 1998 (1998-09-30) & JP 10 175858 A (ITOEN KK), 30 June 1998 (1998-06-30)
- PATENT ABSTRACTS OF JAPAN vol. 1996, no. 8, 30 August 1996 (1996-08-30) & JP 08 109178 A (MITSUI NORIN KK), 30 April 1996 (1996-04-30)
- PATENT ABSTRACTS OF JAPAN vol. 1997, no. 8, 29 August 1997 (1997-08-29) & JP 09 110615 A (MITSUI NORIN KK; SUTEMU KK), 28 April 1997 (1997-04-28)
- JONES R.B., "New Treatments for Chlamydia Trachomatis", AM. J. OBSTET. GYNECOL., Vol. 164, No. 6, Pt. 2, (1991), p. 1789-1793, XP002921224

## Description

The present invention relates to the use of an anti-Chlamydia agent characterized by containing specified tea polyphenol for the preparation of a pharmaceutical composition for the prevention or therapy of a Chlamydia trachomatis infectious disease.

Bacteria belonging to genus Chlamydia are of a spherical or ellipsoidal shape of 0.2 to 1.5 µm and are unique bacteria which are obligate parasites in cells of eucaryotes.

Infectious body, which is an ecotype outside host cells, enters into the vacuoles of host cells and converted therein into particles called reticular structural body due to phagocytosis. The reticular formation increases in number by division and matures into an infectious body in a later stage of infection.

Chlamydia are known as pathogens for trachoma and inclusion conjunctivitis (Chlamydia trachomatis), for parrot disease (C. psittaci), as well as known as pathogens for lung fever (pneumonia), sore throat (pharyngitis), bronchitis, sinusitis, otitis media (C. pneumoniae) or others in child. C. trachomatis is a major pathogenic microorganism for sexually transmitted diseases prevailing worldwide.

For the therapy of Chlamydia infectious diseases, in particular C. trachomatis infectious diseases, oral administration of antibiotics is usually used. In this case, the antibiotics which can be used include doxycycline and mynocycline of tetracycline group, clarithromycin of macrolide group, ofloxacin, tosufloxacin, and sparfloxacin of neuquinolone group, etc. For the therapy, oral administration is usually continued for about 2 weeks. However, in the case of therapy with antibiotics, the problem of side effects is associated. Hence, drugs of tetracycline group and new quinolone group cannot be administered to pregnant women. Moreover, administration of antibiotics is always accompanied by the risk of the emergence of drug-resistant strains.

Accordingly, an object of the present invention is to find an anti-Chlamydia agent which has no side effect nor the fear of emergence of resistant strains out of natural substances and to provide it.

The inventors of the present invention have made intensive investigation in order to achieve the above object and as a result they have found that tea polyphenol contained in tea leaves has a remarkable proliferation inhibiting activity against bacteria belonging to genus Chlamydia, thus completing the present invention based on this finding.

Tea used in the present invention has been used as beverage from old times and has no problem on its safety.

The present invention provides the use of tea polyphenol whereby the tea polyphenol is at least one selected from the group consisting of (+)-catechin, (-)-catechin, (+)-gallocatechin, (+)-epigallocatechin, (+)-gallocatechin gallate, (+)-epigallocatechin gallate, (-) epicatechin, (-) epicatechin gallate, (-)-catechin gallate, (-)-epigallocatechin, (-)-gallocatechin, (-)-epigallocatechin gallate, (-)-gallocatechin gallate, teaflavin monogallate A, teaflavin monogallate B, teaflavin digallate, and free teaflavin for the preparation of a pharmaceutical composition for the prevention or therapy of a Chlamydia trachomatis infectious disease.

In the present invention, tea means leaf, stem, xylem, root, and seed obtained from tea tree (Camellia sinensis) or mixtures of these. Usually, tea leaves for beverages are generally used as a raw material.

Tea leaves for beverages include various kinds depending on the method for their production, for example, fermented tea such as black tea and pooar tea, semi-fermented tea such as oolong tea and paochong tea, and, non-fermented tea such as green tea, and mixtures of these. In the present invention, any of them may be used.

Tea polyphenol which can be used in the present invention includes catechins, that is, (+)-catechin, (-)-catechin,- (+)-gallocatechin, (+)-epigallocatechin, (+)-gallocatechin gallate, (+)-epigallocatechin gallate, (-)-epicatechin, (-)-epicatechin gallate, (-)-catechin gallate, (-)-epigallocatechin, (-)-gallocatechin, (-)-epigallocatechin gallate, (-)-gallocatechin gallate, etc., and teaflavins, that is, teaflavin monogallate A, teaflavin monogallate B, teaflavin digallate or free teaflavin. They are used singly or in combination.

The above tea polyphenol may be commercially available products, for example, those which contain catechins as a major ingredient, such as trade name: Polyphenon 60 (manufactured by Mitsui Norin Co., Ltd., tea polyphenol content: 60% or more), trade name: Polyphenon 30 (manufactured by Mitsui Norin Co., Ltd., tea polyphenol content: 30% or more), trade name: Polyphenon 70S (manufactured by Mitsui Norin Co., Ltd., tea polyphenol content: 70% or more), trade name: Polyphenon E (manufactured by Mitsui Norin Co., Ltd., tea polyphenol content: 80% or more). Those which contain teaflavins as a major ingredient include trade name: Polyphenon TF (manufactured by Mitsui Norin Co., Ltd., composition: 16.8% teaflavin, 19.5% teaflavin monogallate A, 16.1% teaflavin monogallate B, 31.4% teaflavin digallate).

The above defined tea polyphenol used in the present invention is applied to bacteria belonging to the genus Chlamydia, namely C. trachomatis.

In the present invention, upon using the above tea polyphenol, it is combined with a suitable solubilizing agent, suspending agent, base material, or the like and used as a composition in the form of cream, paste, gel, milky lotion, liquid. For example, tea polyphenol and/or tea polyphenol-containing material may be dissolved and/or suspended in purified water, physiological saline, hydrous ethanol and sprayed and/or coated on the affected part such as mucous membrane of respiratory tract. For trachoma, inclusion conjunctivitis, tea polyphenol may be dissolved in purified water or buffer solution and used as eyedroppers or may be mixed with a base material for ointment and used as eyepaste. Also, it is possible to mix tea polyphenol with cream, paste, gel or ointment and to coat the affected part such as epithelium of cervical canal with it.

The bases for cream, paste, gel, and ointment in the present invention include, for example, hydrocarbons such as white vaseline, yellow vaseline, paraffin, liquid paraffin, squalane, and ceresine; higher fatty acids such as lauric acid, stearic acid, myristic acid, palmitic acid, oleic acid, and linolic acid; higher fatty acid alcohol such as stearyl alcohol, oleyl alcohol, lauryl alcohol, cetyl alcohol, and lanolin alcohol; fatty acid esters such as sorbitan sesquioleate, isopropyl myristate, isopropyl palmitate, and glycerin monostearate; waxes such as beeswax, white beeswax, and lanolin; oils and fats such as avocado oil, olive oil, cacao oil, sesame oil, soy bean oil, castor oil, macadamia nut oil, mink oil, yolk oil, beef tallow, and lard; high molecular compounds such as gum arabi, tragacanth gum, guar gum, karaya gum, dextrin, gelatin, carrageenan, shellac, rosin, casein, sodium carboxymethylcellulose, methylcellulose, ethylcellulose, sodium alginate, nitrocellulose, polyvinyl alcohol, polyvinylpyrrolidone, sodium polyacrylate, polyvinyl methyl ether, lauromacrogol, polyamide resins, and silicone oil, and one or more of these may be selected appropriately and used.

To the above preparations can be added humectants such as glycerin, propylene glycol, 1,3-butylene glycol, polyethylene glycol, sodium dl-pyrrolidonecarboxylate, sodium lactate, sorbitol, sodium hyaluronate; inorganic substances such as bentonite, kaolin, zinc oxide, and titanium oxide; stabilizers such as methyl paraoxybenzoate, ethyl paraoxybenzoate, propyl paraoxybenzoate, and butyl paraoxybenzoate; antiseptics such as benzalkonium chloride, benzethonium chloride, citric acid, sodium citrate, paraoxybenzoic acid, and boric acid; surfactants such as polyoxyethylene-hydrated castor oil, and known percutaneous absorption promoter, if desired.

In these cases, the concentration of tea polyphenol in the drug may vary depending on the symptom and age of patients, site of use, method of use and is not limited particularly. Usually, when used in the form of liquid or milky lotion, the concentration of tea polyphenol in the drug is 0.2 to 50 mg/ml, preferably 1.6 to 10 mg/ml. When used in the form of cream, paste, gel or ointment, the concentration of tea polyphenol in the drug is 0.2 to 200 mg/g, preferably 10 to 100 mg/g.

When using the tea polyphenol as defined above it is desirable to continue the therapy for a certain period of time, e.g., 2 to 4 weeks, taking into consideration the unique growth cycle of Chlamydia and prevention of recurrence. Although the frequency of use of the agent during that time may vary depending on the factors, such as symptom of the patient, site of use, method of use, concentration of tea polyphenol used, it is possible to continue daily use of 1 to 10 times a day.

The tea polyphenol used in the present invention is highly safe so that it can be used for preventive purposes.

Hereafter, the present invention will be described more specifically by examples.

### Example 1

As test Chlamydia was used C. trachomatis strain. Chlamydia capable of forming 10⁴ inclusion bodies was incubated at 37°C for 30 minutes, 60 minutes or 90 minutes in a SPG (sucrose phosphate glutamate) solution containing tea polyphenol of various concentrations (trade name: Polyphenon 70S manufactured by Mitsui Norin Co., Ltd., catechin content: (-)-epigallocatechin 18.3%, (-)-epicatechin 8.6%, (-)-epigallocatechin gallate 35.9%, (-)-epicatechin gallate 11.2%, (-)-gallocatechin gallate 3.5%). As a control, Chlamydia was incubated similarly in SPG solution containing no tea polyphenol.

After completion of the incubation, each solution was inoculated onto HeLa 229 cell cultivated by monolayer culture and adsorbed by centrifugation at 1,500 rpm for 60 minutes. Thereafter, the inoculum was removed and culture medium for Chlamydia (Eagle Minimum Essential Culture Medium containing 1 µg/ml cycloheximide) was added and cultivation was performed at 37°C for 72 hours. After completion of cultivation, the culture medium was removed and the cells were fixed with methanol and then stained with fluorescein isothiocyanate (FITC)-labeled anti-C. trachomatis monoclonal antibody, followed by counting the number of inclusion bodies of

### C. trachomatis.

The results obtained are shown in Table 1. In the table, the number of inclusion bodies in each treatment is indicated as a relative value with respect to the number of inclusion bodies in the control. As will be apparent from the table, in each group the number of C. trachomatis inclusion bodies decreased except when incubated for 30 minutes after addition of 0.2 mg/ml of tea polyphenol. In particular, when incubated for 90 minutes, addition of 1.6 mg/ml or more of tea polyphenol completely inhibited the growth of C. trachomatis.

**Table 1**

| Incubation time (minute) | Concentration of Tea Polyphenol (mg/ml) | | | | | | |
|---|---|---|---|---|---|---|---|
| | 0 | 0.2 | 0.4 | 0.8 | 1.6 | 3.2 | 6.4 |
| 30 | 1.00 | 1.00 | 0.58 | 0.34 | 0.18 | 0.10 | 0.05 |
| 60 | 1.00 | 0.87 | 0.41 | 0.18 | 0.06 | 0.02 | 0.02 |
| 90 | 1.00 | 0.55 | 0.07 | 0.03 | 0 | 0 | 0 |

### Example 2

As test Chlamydia was used C. trachomatis strain. Chlamydia capable of forming 10⁴ inclusion bodies was incubated at 37°C for 90 minutes in a SPG solution containing (-)-epicatechin gallate or (-)-epigallocatechin gallate in various concentrations.

After completion of the incubation, each solution was inoculated onto Hela 229 cell cultivated by monolayer culture and adsorbed by centrifugation at 1,500 rpm for 60 minutes. Thereafter, the inoculum was removed and culture medium for Chlamydia (Eagle Minimum Essential Culture Medium containing 1 µg/ml cycloheximide) was added and cultivation was performed at 37°C for 72 hours. After completion of cultivation, the culture medium was removed and the cells were fixed with methanol and then stained with FITC-labeled anti-C. trachomatis monoclonal antibody, followed by counting the number of inclusion bodies of C. trachomatis.

As a result, it was found that (-)-epicatechin gallate and (-)-epigallocatechin gallate completely inhibited the growth of C. trachomatis in concentrations of 0.8 mg/ml and 1.6 mg/ml, respectively.

### Example 3

Anti-Chlamydia agents were prepared in the following formulations. The compositional analysis values of "Polyphenon E" used as tea polyphenol are as follows.

| Composition of "Polyphenon E" | |
|---|---|
| (-)-Epigallocatechin | 12% |
| (-)-Epicatechin | 9% |
| (-)-Epigallocatechin gallate | 53% |
| (-)-Gallocatechin gallate | 6% |
| (-)-Epicatechin gallate | 4% |

| Prescription Example 1 | |
|---|---|
| Zinc oxide | 200 g |
| Liquid paraffin | 30 g |
| White beeswax | 32.5 g |
| Sorbitan sesquioleate | 13 g |
| White vaseline . | 604.5 g |
| "Polyphenon E" | 120 g |
| Total amount | 1,000 g |

| Prescription Example 2 | |
|---|---|
| White vaseline | 400 g |
| Cetanol | 100 g |
| White beeswax | 50 g |
| Sorbitan sesquioleate | 50 g |
| Lauromacrogol | 5 g |
| Ethyl paraoxybenzoate (or methyl paraoxybenzoate) | 1 g |
| Butyl paraoxybenzoate (or propyl paraoxybenzoate) | 1 g |
| "Polyphenon E" | 120 g |
| Purified, water | 273 g |
| Total amount | 1,000 g |

| Prescription Example 3 | |
|---|---|
| White vaseline | 250 g |
| Stearyl alcohol | 200 g |
| Propylene glycol | 120 g |
| Polyoxyethylene-hydrated castor oil 60 | 40 g |
| Glycerin monostearate | 10 g |
| Methyl paraoxybenzoate | 1 g |
| Propyl paraoxybenzoate | 1 g |
| "Polyphenon E" | 120 g |
| Purified water | 258 g |
| Total amount | 1,000 g |

| Prescription Example 4 | |
|---|---|
| Beeswax | 330 g |
| Vegetable oil | 550 g |
| "Polyphenon E" | 120 g |
| Total amount | 1,000 g |

| Prescription Example 5 | |
|---|---|
| White beeswax | 50 g |
| Sorbitan sesquioleate | 20 g |
| White vaseline | 810 g |
| "Polyphenon E" | 120 g |
| Total amount | 1,000 g |

| Prescription Example 6 | |
|---|---|
| Macrogol (polyethylene glycol) 4,000 | 440 g |
| Macrogol (polyethylene glycol) 400 | 440 g |
| "Polyphenon E" | 120 g |
| Total amount | 1,000 g |

| Prescription Example 7 | |
|---|---|
| Stearic acid | 200 g |
| Potassium hydroxide | 13 g |
| Glycerin | 100 g |
| Methyl paraoxybenzoate | 1 g |
| Propyl paraoxybenzoate | 1 g |
| "Polyphenon E" | 120 g |
| Purified water | 565 g |
| Total amount | 1,000 g |

| Prescription Example 8 | |
|---|---|
| Stearic acid | 150 g |
| Isopropyl palmitate | 20 g |
| Lanolin | 10 g |
| Sorbitol | 56 g |
| Potassium hydroxide | 10 g |
| Methyl paraoxybenzoate | 1 g |
| Propyl paraoxybenzoate | 1 g |
| "Polyphenon E" | 120 g |
| Purified water | 632 g |
| Total amount | 1,000 g |

| Prescription Example 9 | |
|---|---|
| Stearic acid | 180 g |
| Liquid paraffin | 20 g |
| Lanolin | 5 g |
| Sorbitan sesquioleate | 20 g |
| Potassium hydroxide | 8 g |
| Sorbitol | 35 g |
| Methyl paraoxybenzoate | 1 g |
| Propyl paraoxybenzoate | 1 g |
| "Polyphenon E" | 120 g |
| Purified water | 610 g |
| Total amount | 1,000 g |

| Prescription Example 10 | |
|---|---|
| Boric acid | 20 mg |
| Methyl paraoxybenzoate | 0.26 mg |
| Propyl paraoxybenzoate | 0.14 mg |
| "Polyphenon E" | 250 mg |
| Purified water | total amount 1,000 ml |

| Prescription Example 11 | |
|---|---|
| Sodium dihydrogen phosphate anhydride | 5.6 mg |
| Sodium hydrogen phosphate anhydride | 2.84 mg |
| Methyl paraoxybenzoate | 0.26 mg |
| Propyl paraoxybenzoate | 0.14 mg |
| "Polyphenon E" | 250 mg |
| Purified water | total amount 1,000 ml |

| Prescription Example 12 | |
|---|---|
| Methyl paraoxybenzoate | 0.26 mg |
| Propyl paraoxybenzoate | 0.14 mg |
| "Polyphenon E" | 250 mg |
| Purified water | total amount 1,000 ml |

According to the present invention, a preventive and therapeutic medicine which is effective on Chlamydia infectious diseases and highly safe is provided. This medicine has no side effect nor the risk of emergence of resistant strains unlike the case where antibiotics are administered.

## Claims

1. Use of tea polyphenol whereby the tea polyphenol is at least one selected from the group consisting of (+)-catechin, (-)-catechin, (+)-gallocatechin, (+)-epigallocatechin, (+)-gallocatechin gallate, (+)-epigallocatechin gallate, (-)-epicatechin, (-)-epicatechin gallate, (-)-catechin gallate, (-)-epigallocatechin, (-)-gallocatechin, (-)-epigallocatechin gallate, (-)-gallocatechin gallate, teaflavin monogallate A, teaflavin monogallate B, teaflavin digallate, and free teaflavin for the preparation of a pharmaceutical composition for the prevention or therapy of a Chlamydia trachomatis infectious disease.

2. Use as claimed in claim 1, wherein the composition is in the form of cream, paste, gel, ointment, milky lotion, solution or suspension.

3. Use as claimed in claim 1 or 2, wherein the tea polyphenol in the composition is in a concentration of 0.2 to 50 mg/ml when used in the form of liquid or milky lotion, or 0.2 to 200 mg/g when used in the form of cream, paste, gel or ointment.

## Patentansprüche

1. Verwendung von Tee-Polyphenol, wobei das Tee-Polyphenol mindestens ein Mitglied ist, das ausgewählt ist aus der Gruppe bestehend aus (+)-Catechin, (-)-Catechin, (+)-Gallocatechin, (+)-Epigallocatechin, (+)-Gallocatechingallat, (+)-Epigallocatechingallat, (-)-Epicatechin, (-)-Epicatechingallat, (-)-Catechingallat, (-)-Epigallocatechin, (-)-Gallocatechin, (-)-Epigallocatechingallat, (-)-Gallocatechingallat, Teeflavin-Monogallat A, Teeflavin-Monogallat B, Teeflavin-Digallat und freiem Teeflavin, für die Herstellung eines Arzneimittels zur Prävention oder Therapie einer Chlamydia trachomatis-Infektionserkrankung.

2. Verwendung nach Anspruch 1, wobei das Mittel in Form einer Creme, Paste, eines Gels, einer Salbe, milchigen Lotion, Lösung oder Suspension vorliegt.

3. Verwendung nach Anspruch 1 oder 2, wobei das Tee-Polyphenol in dem Mittel in einer Konzentration von 0,2 bis 50 mg/ml vorliegt, wenn es in Form von Flüssigkeit oder milchiger Lotion verwendet wird, oder 0,2 bis 200 mg/g, wenn es in Form einer Creme, Paste, eines Gels oder einer Salbe verwendet wird.

## Revendications

1. Utilisation de polyphénol de thé, dans laquelle le polyphénol de thé est un au moins choisi dans le groupe constitué de (+)-catéchine, (-)-catéchine, (+)-gallocatéchine, (+)-epigallocatéchine, (+)-gallocatéchine gallate, (+)-epigallocathéchine gallate, (-)-epicatéchine, (-)-epicatéchine gallate, (-)-catéchine gallate, (-)-epigallocatéchine, (-)-gallocatéchine, (-)-epigallocatéchine gallate, (-)-gallocatéchine gallate, monogallate A de flavine de thé, monogallate B de flavine de thé, digallate de flavine de thé, et flavine de thé libre, pour la préparation d'une composition pharmaceutique pour la prévention ou la thérapie d'une maladie infectieuse due à Chlamydia trachomatis.

2. Utilisation telle que revendiquée dans la revendication 1, dans laquelle la composition se présente sous la forme d'une crème, d'une pâte, d'un gel, d'un onguent, d'une lotion à base de lait, d'une solution ou d'une suspension.

3. Utilisation telle que revendiquée dans la revendication 1 ou 2, dans laquelle le polyphénol de thé est présent dans la composition à une concentration de 0,2 à 50 mg/ml lorsqu'il est utilisé sous la forme d'un liquide ou d'une lotion à base de lait, ou de 0,2 à 200 mg/g lorsqu'il est utilisé sous la forme d'une crème, pâte, gel ou onguent.
